(19) Europäisches Patentamt
European Patent Office
Office européen des brevets

(11) Numéro de publication : **0 489 670 A1**

# (12) DEMANDE DE BREVET EUROPEEN

(21) Numéro de dépôt : **91420434.2**

(22) Date de dépôt : **05.12.91**

(51) Int. Cl.[5] : **C07C 49/84,** C07C 43/23, C07C 69/007, C07C 205/45, C07C 225/22, C07C 205/37, C07C 205/60, A01N 35/04, A01N 31/16

(30) Priorité : **06.12.90 FR 9015529**

(43) Date de publication de la demande : **10.06.92 Bulletin 92/24**

(84) Etats contractants désignés : **AT BE CH DE DK ES FR GB GR IT LI LU NL SE**

(71) Demandeur : **RHONE-POULENC AGROCHIMIE**
**14-20, rue Pierre Baizet**
**F-69009 Lyon (FR)**

(72) Inventeur : **Gadras, Alain**
**Les Hauts de l'Ile Barbe, 208 Rue de St. Cyr**
**F-69009 Lyon (FR)**
Inventeur : **Peignier, Raymond**
**81 Bis Chemin de Vassieux**
**F-69300 Caluire (FR)**
Inventeur : **Tadj, Fatemeh**
**17, Place Maréchal Lyautey**
**F-69006 Lyon (FR)**

(74) Mandataire : **Brachotte, Charles et al**
**RHONE-POULENC AGROCHIMIE P.I.D. BP 9163**
**F-69263 Lyon Cedex 09 (FR)**

## (54) 2,6-Alkoxyphényl alkylcétone et dérivés herbicides.

(57) Herbicides 2,6-dialkoxy phenyle Herbicides de formule :

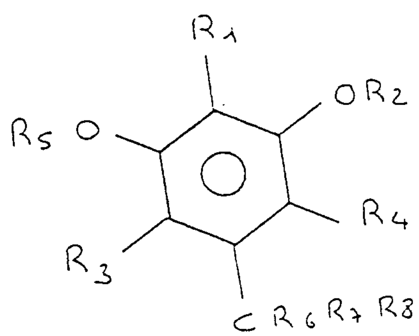

dans laquelle :
$R_1$ est un groupe C(=O)-$R_9$ X est O ou S ; $R_9$ étant un radical hydrocarbyle éventuellement substitué ; CROHR$_9$ ou CHR$_9$OR$_{14}$, $R_9$ ayant l'une des significations de $R_9$, $R_{14}$ étant un radical hydrocarbyle éventuellement substitué ou CHR$_9$OC(=O)R$_{15}$, $R_{15}$ étant un radical hydrocarbyle éventuellement substitué ;-
C(=O)R$_{16}$, $R_{16}$ étant OR$_{13}$ ou SR$_{13}$, $R_{13}$ étant un radical hydrocarbyle éventuellement substitué.
$R_2$, $R_5$, identiques ou différents sont des radicaux hydrocarbyles éventuellement substitués.
$R_3$, $R_4$ identiques ou différents sont un atome d'hydrogène, ou des radicaux $C_1$-$C_6$ alkyles éventuellement substitués ou un des radicaux $R_3$ ou $R_4$ peuvent être le radical nitro ou amino, $R_6$, $R_7$, $R_6$ identiques ou différents sont des radicaux $C_1$-$C_6$ alkyle $C_3$-$C_7$ cyclo alkyle éventuellement substitués, et les sels acceptables en agriculture de ces composés.

EP 0 489 670 A1

L'invention est relative à de nouveaux composés, à leur utilisation à titre d'herbicides notamment sous forme de composition herbicide, à un procédé de contrôle des mauvaises herbes à l'aide de ces composés ou de ces compositions, aux procédés de synthèse des composés selon l'invention, aux composés éventuellement utilisables dans les procédés de synthèse selon l'invention.

Un objet de la présente invention est donc de proposer des composés utiles comme herbicides. Un autre objet de la présente invention est de proposer des composés utiles en pré émergence comme herbicides.

Un autre objet de la présente invention est de proposer des composés utiles en pré émergence comme herbicides sélectifs de nombreuses cultures dicotylédones (notamment soja, colza, tournesol, coton) et de nombreuses cultures monocotylédones (blé, riz, maïs).

Ces buts sont partiellement ou totalement atteints au moyen des composés selon l'invention. L'invention concerne en premier lieu de façon générale des composés qui répondent à la formule (I):

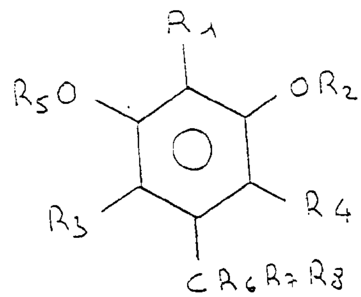

I

dans laquelle : $R_1$ est un radical C(= X)-$R_9$, X est O ou S, $R_9$ étant un atome d'hydrogène ou un groupe hydrocarbyle éventuellement substitué ; ou un groupe -$CR_9R_{9a}0R_{14}$, $R_{9a}$ ayant l'une des significations de $R_9$, $R_{14}$ étant un radical hydrocarbyle éventuellement substitué ou un radical hydrocarbyle éventuellement substitué carbonyle ; ou un groupe C(=0)-$R_{16}$, $R_{16}$ étant $OR_{13}$ ou $SR_{13}$, $R_{13}$ étant un radical hydrocarbyle éventuellement substitué,

$R_2$, $R_5$, identiques ou différents sont des groupes hydrocarbyles éventuellement substitués,

$R_3$, $R_4$ identiques ou différents sont un atome d'hydrogène, les radicaux $C_1$-$C_6$ alkyles éventuellement substitués, les radicaux nitro, amino à la condition qu'un seul des radicaux $R_3$ ou $R_4$ puissent signifier nitro ou amino simultanément, $R_5$, $R_7$, $R_5$ identiques ou différents sont des radicaux $C_1$-$C_6$ alkyle, $C_3$-$C_7$ cycloalkyle éventuellement substitués.

L'invention comprend également les sels acceptables en agriculture des composés de formule (I) dans le cas où $R_3$ ou $R_4$ correspondent au radical amino, ou quand le composé comporte un groupe carboxy. Comme sels on peut citer les chlorhydrates, hydrogenosulfates notamment, et les sels de sodium.

Un radical hydrocarbyle est de façon bien connue un reste hydrocarboné aliphatique saturé ou insaturé ou aromatique ou alicyclique comprenant éventuellement un ou plusieurs hétéroatomes.

De façon plus particulière l'invention est définie ainsi :

Composés de formule (I) :

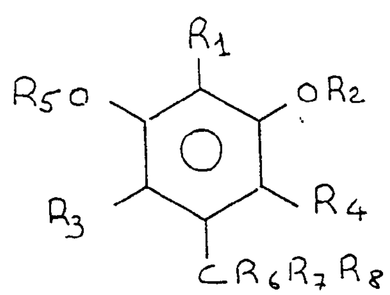

(I)

dans laquelle :

$R_1$ représente :

– un groupe -C(=X)-$R_9$, X = 0 ou S et $R_9$ étant un atome d'hydrogène ou un radical $C_1$-$C_6$ alkyle éventuellement substitué par un ou plusieurs atomes d'halogène (de préférence le radical trifluorométhyle) ou radicaux $C_1$-$C_4$ alkoxy, $C_1$-$C_4$ alkylthio ; un radical $C_2$-$C_6$ alcynyle ou $C_2$-$C_6$ alcényle, lesdits radicaux étant

éventuellement substitués de la même manière que le groupement $C_1$-$C_6$ alkyle ; un radical di ($C_1$-$C_3$) alkylamino ($C_1$-$C_3$) alkyl, di ($C_1$-$C_3$) alkylamino $C_1$-$C_6$ alkylènyle, amino $C_1$-$C_6$ alkylènyle ou mono ($C_1$-$C_3$) alkylamino $C_1$-$C_6$ alkylènyle ; un radical $C_3$-$C_6$ cycloalkyle (de préférence cyclopropyle) substitué ou non par un ou plusieurs atomes d'halogène ou par un ou plusieurs radicaux choisis parmi les radicaux $C_1$-$C_6$ alkyle (de préférence méthyle), $C_1$-$C_6$ haloalkyle, $C_1$-$C_6$ alkoxy, $C_1$-$C_6$ thioalkoxy ; un radical $C_6$-$C_{10}$ aryle (notamment phényle) substitué ou non par un ou plusieurs atomes d'halogène ou par un ou plusieurs radicaux choisis parmi les radicaux $C_1$-$C_6$ alkyle (de préférence méthyle), $C_1$-$C_6$ haloalkyle, $C_1$-$C_6$ alkoxy, $C_1$-$C_6$ thioalkoxy ; un radical $C_7$-$C_{11}$ aralkyle (notamment benzyle, phénethyle) éventuellement substitué de la même manière que le radical $C_6$-$C_{10}$ aryle ; un radical hétéroaryle ayant 5 ou 6 atomes dont 1 à 3 hétéroatomes choisis parmi le soufre, l'azote, l'oxygène, notamment les 2- ou 3- thiényle, les 2- ou 3- furyle, les pyrolyles, les pyrrazolyles, les pyridinyles, les imidazolyles, les triazolyles, les pyrimidinyles, les pyridazinyles, les 2-, 3-, 4-, 5-, oxazolyles ou thiazolyles,

– un groupe

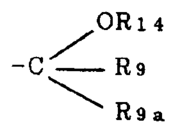

dans lequel $R_{9a}$ a l'une des significations mentionnées pour $R_9$ (de préférence avec $R_9$ ou $R_{9a}$ représentant l'atome d'hydrogène), $R_{14}$ forme, ensemble avec $R_9$ ou $R_{9a}$, une chaîne $C_1$-$C_6$ alkylène ou $R_{14}$ est l'atome d'hydrogène, un radical $C_1$-$C_6$ alkyle éventuellement substitué par un ou plusieurs atomes d'halogène (de préférence le radical trifluorométhyle) ou radicaux $C_1$-$C_4$ alkoxy, $C_1$-$C_4$ alkylthio ; un radical $C_2$-$C_6$ alcynyle ou $C_2$-$C_6$ alcényle lesdits radicaux étant éventuellement substitués de la même manière que le groupement $C_1$-$C_6$ alkyle ; un radical $C_3$-$C_6$ cycloalkyle (de préférence cyclopropyle) substitué ou non par un ou plusieurs atomes d'halogène ou par un ou plusieurs radicaux choisis parmi les radicaux $C_1$-$C_6$ alkyle (de préférence méthyle), $C_1$-$C_6$ haloalkyle, $C_1$-$C_6$ alkoxy, $C_1$-$C_6$ thioalkoxy ; un radical $C_6$-$C_{10}$ aryle (notamment phényle) substitué ou non par un ou plusieurs atomes d'halogène ou par un ou plusieurs radicaux choisis parmi les radicaux $C_1$-$C_6$ alkyle (de préférence méthyle), $C_1$-$C_6$ haloalkyle, $C_1$-$C_6$ alkoxy, $C_1$-$C_6$ thioalkoxy ; un radical $C_7$-$C_{11}$ aralkyle (notamment benzyle, phénethyle) éventuellement substitué de la même manière que le radical $C_6$-$C_{10}$ aryle ; un radical hétéroaryle ayant 5 ou 6 atomes dont 1 à 3 hétéroatomes choisis parmi le soufre, l'azote, l'oxygène, notamment les 2- ou 3- thiényle, les 2- ou 3- furyle, les pyrolyles, les pyrrazolyles, les pyridinyles, les imidazolyles, les triazolyles, les pyrimidinyles, les pyridazinyles, les 2-, 3-, 4-, 5-, oxazolyles ou thiazolyles.

$R_{14}$ est encore un radical C(=O)-$R_{15}$ dans lequel $R_{15}$ représente une chaine $C_1$-$C_6$ alkyle, éventuellement substituée par un ou plusieurs atomes d'halogène (de préférence le radical trifluorométhyle) ou radicaux $C_1$-$C_4$ alkoxy, $C_1$-$C_4$ alkylthio, $C_1$-$C_4$ alkylsulfonyle, $C_1$-$C_4$ alkylsulfinyle ; un radical $C_2$-$C_6$ alcynyle ou $C_2$-$C_6$ alcényle lesdits radicaux étant éventuellement substitués de la même manière que le groupement $C_1$-$C_6$ alkyle ; un radical $C_3$-$C_6$ cycloalkyle substitué ou non par un ou plusieurs atomes d'halogène ou par un ou plusieurs radicaux choisis parmi les radicaux $C_1$-$C_6$ alkyle (de préférence méthyle), $C_1$-$C_6$ haloalkyle, $C_1$-$C_6$ alkoxy, $C_1$-$C_6$ thioalkoxy ; un radical $C_6$-$C_{10}$ aryle (notamment phényle) substitué ou non par un ou plusieurs atomes d'halogène ou par un ou plusieurs radicaux choisis parmi les radicaux $C_1$-$C_6$ alkyle (de préférence méthyle), $C_1$-$C_6$ haloalkye, $C_1$-$C_6$ alkoxy, $C_1$-$C_6$ thioalkoxy ; un radical $C_7$-$C_{11}$ aralkyle (notamment benzyle, phénethyle) éventuellement substitué de la même manière que le radical $C_6$-$C_{10}$ aryle ; un radical hétéroaryle ayant 5 ou 6 atomes dont 1 à 3 hétéroatomes choisis parmi le soufre, l'azote, l'oxygène, notamment les 2- ou 3- thiényle, les 2- ou 3- furyle, les pyrolyles, les pyrrazolyles, les pyridinyles, les imidazolyles, les triazolyles, les pyrimidinyles, les pyridazinyles, les 2-, 3-, 4-, 5-, oxazolyles ou thiazolyles,

– un grouye -C(=O)-$R_{16}$ dans lequel $R_{16}$ est $OR_{13}$ ou $SR_{13}$, avec $R_{13}$, qui peut être un atome d'hydrogène ou un radical $C_1$-$C_6$ alkyle, $C_2$-$C_6$ alcényle ou $C_2$-$C_6$ alcynyle, un radical $C_6$-$C_{10}$ aryle (notamment phényle), $C_7$-$C_{11}$ aralkyle (notamment benzyle), lesdits radicaux étant substitués comme précédemment ou hétéroaryle ayant de 5 à 6 atomes dont 1 à 3 hétéroatomes choisis parmi l'azote, le soufre, l'oxygène,

$R_2$, $R_5$, identiques ou différents, représentent un groupe $C_1$-$C_6$ alkyle, $C_2$-$C_6$ alcènyle, $C_2$-$C_6$ alcynyle, ces deux radicaux étant éventuellement substitués de la même facon que le radical $C_1$-$C_6$ alkyle indiqué pour le radical $R_9$,

$R_3$, $R_4$, identiques ou différents, représentent l'atome d'hydrogène ou un groupe $C_1$-$C_6$ alkyle (méthyle, éthyle, n-propyle, i-propyle, etc...), un radical nitro ou amino à la condition que un seul des radicaux $R_3$, $R_4$ puissent signifier nitro ou amino simultanément,

$R_6$, $R_7$, $R_8$ identiques ou différents représentent un radical $C_1$-$C_6$ alkyle, un radical $C_3$-$C_7$ cycloalkyle. Au sens du présent texte les radicaux alkyle, alcényle, alcynyle, alkoxy, alkylthio etc... peuvent être linéaires ou ramifiés et le préfixe halo signifie que les radicaux sont mono ou polyhalogénés. En vue des applications herbicides ou de leur facilité d'obtention, il a été trouvé que les composés des variantes indiquées ci-après prises ou non en combinaison avec la définition de l'invention sus indiquée était préférés.

– X est l'atome d'oxygène
– le radical $CR_6R_7R_8$ est le tertiobutyle
– $R_3$ et $R_4$ sont l'atome d'hydrogène ou l'un des groupes $R_3$ ou $R_4$ est l'atome d'hydrogène et l'autre est le radical nitro,
– $R_2$ et $R_5$ identiques ou différents correspondent au radical $C_1$-$C_3$ alkyle et, de préférence,
– $R_2$ et $R_5$ sont le radical méthyle
– $R_1$ est le radical C(= 0)-$R_9$ dans lequel $R_9$ est un radical $C_1$-$C_4$ alkyle.
– $R_1$ est le radical $HCOHR_9$ dans lequel $R_9$ est un radical $C_1$-$C_4$ alkyle.
– $R_1$ est le radical $HCOR_{14}R_9$, $R_9$ étant un radical $C_1$-$C_4$ alkyle, $R_{14}$ étant un radical $C_1$-$C_4$ alkyle
– $R_1$ est le radical-$HR_9COC(=O)R_{15}$, $R_{15}$ étant un radical $C_1$-$C_4$ alkyle.

Les composés selon l'invention de formule (II) :

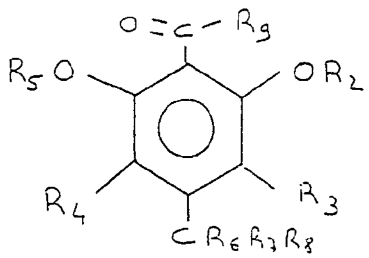

où les substituants $R_2$, $R_8$, $R_6$, $R_7$, $R_8$ ont la même signification que précédemment et R3, R4 sont différents des radicaux amino, nitro peuvent être préparés à partir d'un dérivé de formule (III)

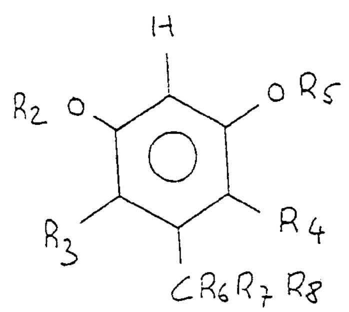

par une réaction de substitution électrophile du type FRIEDEL et CRAFTS au moyen du composé $R_9COHal$ (Hal étant un atome d'halogène, notamment le chlore) en présence d'un catalyseur du type acide de LEWIS (notamment le trichlorure d'aluminium) dans un solvant aromatique ou halogéné (notamment le dichlorométhane) selon la méthode décrite par exemple dans Textbook of Practical Organic Chemistry, VOGEL'S, John Wiley & Sons, Inc., New-York (1978) p 770.

Une autre méthode de préparation des composés de formule (II) consiste à partir d'un dérivé de formule (III) sur lequel est effectuée une ortholithiation selon la procédure décrite notamment dans Préparative Polar Organometallic Chemistry Springer-Verlag, 1 (1987) p 204. Brandsma et Verkruysse ; puis de faire réagir sur le sel de lithium formé un chlorure d'acide $R_9COCl$ comme décrit dans Indian Journal of Chemistry [B] 14 (1976) p 168 Bhide et coll.

Le composé de formule (III) dans lequel $R_8$, $R_7$, $R_8$ représentent le radical méthyle peut être préparé en 6 étapes à partir du 2,6 dinitro 4-terbutyl-acétanilide selon la méthode décrite dans Chemiche Berichte 98 (9) (1965) pp 2774-2796 H. MUSSO et D. BORMANN.

Le 2,6-dinitro 4-terbutyl acétanilide peut lui-même être préparé en 3 étapes à partir de la 4-terbutylaniline, obtenue de manière connue, selon la méthode décrite dans Journal of The Chemical Society [London] (1958) pp 120-126 F.BELL.

Le composé de formule (III) en général peut être obtenu à partir d'un composé de formule IV

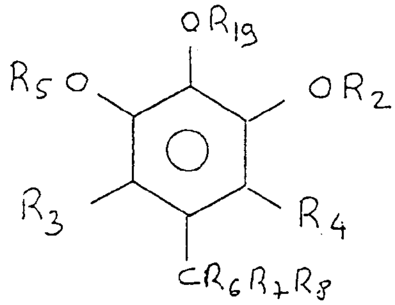

IV

dans laquelle $R_{19}$ est le radical méthyle par réaction avec un métal réducteur (notamment le sodium ou le potassium) selon le procédé décrit dans Journal of Chemical Society, Chemical Communication (1987) pp 1549-1550 U. AZZENA et coll.

Une variante consiste à partir d'un composé de formule IV dans lequel $R_{19}$ est le radical P(=0) $(0Et)_2$ et de le faire réagir avec du lithium en présence d'ammoniac selon la méthode décrite dans Journal of Organic Chemistry, 42 (2) (1977) pp 344-346 S.J. DOMINIANNI et coll.

Les composés de formule IV dans lesquels $R_{19}$ est le radical méthyle peuvent être préparés à partir du composé de formule V:

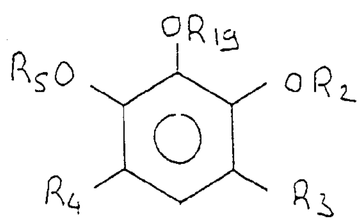

V

dans lequel $R_{19}$ est le radical méthyle par réaction avec un groupe $HalCR_6R_7R_8$ où Hal représente un atome d'halogène (notamment le chlore) en présence d'un catalyseur de type acide de Lewis (notamment le chlorure ferrique) comme décrit dans Textbook of Practical Organic Chemistry VOGEL'S, John Wiley & Sons, Inc., New-York (1978) p 606.

Les composés de formule IV dans lesquels $R_{19}$ est le radical méthyle peuvent également être préparés à partir du composé V dans lequel $R_{19}$ est l'atome d'hydrogène par réaction avec un groupe $HOCR_6R_7R_8$ en présence d'un acide organique ou minéral comme décrit dans Journal of Organic Chemistry, 42 (2) (1977) p 345 SJ DOMINIANNI et coll ou dans Journal of The Chemical Society (c) (1968) p 1442 F.R. HEWGILL et coll. Les composés de formule V sont obtenus de manière connue.

Les composés de formule (II), dans lesquels $R_3$ ou $R_4$ est le radical nitro, peuvent être préparés à partir d'un composé (II) précedemment décrit dans lequel un des radicaux $R_3$, $R_4$ au moins représentent un atome d'hydrogène par une réaction de nitration, à une température allant de -10° C à +100° C comme décrit dans Organic Synthesis, IV, p 364 HP SCHULTZ.

La réduction de la fonction nitro en amino peut être réalisée de manière connue comme décrit dans Advanced Organic Chemistry, Mac Graw Hill, Inc J. March.

Les composés de formule (I) dans lesquels

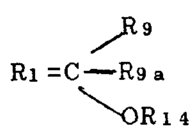

avec $R_{14}$ = H et les autres substituants ayant la même signification que précédemment, peuvent être préparés de manière connue par réduction d'un dérivé de formule (II) ou par addition d'un organométallique $R_{9a}M$ (dans lequel M = MgHal ou Li) sur le dérivé de formule (II) précité selon les méthodes décrites dans Advanced Organic Chemistry, Mc. Graw. Hill, Inc J. March.

L'alcool formé peut ensuite être substitué de manière connue par action d'une base dans un solvant polaire protique ou aprotique suivi d'une réaction avec un composé de formule $R_{14}$ Hal à une température comprise

entre 0°C et 100°C. (Hal représente un atome d'halogène).

L'halogène peut être situé sur le radical $R_9$ ou $R_{9a}$ et la réaction conduit dans ce cas à la formation du dérivé cyclique.

L'alcool formé peut également être estérifié par $R_{15}$-CO-Cl ou l'anhydride correspondant en présence d'un accepteur d'acide dans un solvant inerte.

Les composés de formule (I) dans lesquels

$$R_1 = -C(=0)R_{16}$$

peuvent être préparés, via l'acide intermédiaire, à partir d'un dérivé du type (III) par une réaction d'ortho lithiation comme décrit dans les méthodes générales de préparation de (I) ci-dessus, puis de faire réagir l'anhydride carbonique sur l'anion formé comme décrit par exemple dans Journal of Organic Chemistry, 36 (1971) p 1843 J.G. Lombardino.

L'acide formé peut ensuite être converti en ester selon des méthodes bien connues.

Les exemples suivant illustrent l'invention. Les exemples 1 et 2 illustrent la préparation d'intermédiaires pour atteindre les composés de formule (I).

Exemple 1

Préparation du 1, 2, 3-triméthoxy 5-terbutyl benzène

Dans un ballon tricol de 2000 ml équipé d'une ampoule de coulée de 250 ml, d'un réfrigérant et muni d'une agitation mécanique, on introduit 450 ml de 1,2-dichloroéthane, 120 g (0,71 mole) de 1, 2, 3-triméthoxybenzène et 11,5 g (0,071 mole) de chlorure ferrique.

On coule goutte à goutte 92 ml (0,85 mole) de 2-chloro-2-méthyl propane à température ambiante puis le milieu est chauffé à 50°C pendant 15 h.

Le milieu est ensuite hydrolysé dans un bain de glace avec 250 ml d'acide chlorydrique 0,1 N, extrait avec 200 ml de chlorure de methylène, lavé avec 300 ml d'eau et séché sur sulfate de magnésium.

Après élimination sous vide des solvants, l'huile brune obtenue est introduite à nouveau dans le ballon de 2000 ml avec 83 g (0,6 mole) de carbonate de potassium, 57 ml (0,6 mole) de sulfate de méthyle et 600 ml d'acétonitrile. Le milieu est porté au reflux de l'acétonitrile pendant 12 h.

Après retour à température ambiante, le carbonate de potassium est filtré et le filtrat concentré sous vide. Le résidu obtenu est repris avec 150 ml d'éther et hydrolysé avec 200 ml d'eau. La phase aqueuse est extraite avec 200 ml d'éther puis les phases éthérées rassemblées lavées avec 250 ml d'eau, séchées sur sulfate de magnésium puis concentrées. L'huile brune obtenue est distillée sous vide au moyen d'une colonne vigreux.

On obtient (1) sous la forme d'un liquide incolore qui présente une température d'ébullition de 94,1-94,3°C sous 0,4 torr

Masse obtenue : 124 g Rendement : 78 %

Exemple (2)

Préparation de 1,3-dimethoxy 5-Terbutylbenzène

Dans un ballon tricol de 2000 ml équipé d'une ampoule de coulée isobare de 250 ml, d'un réfrigérant et muni d'une agitation mécanique, on introduit sous un courant d'azote 800 ml de tetrahydrofuranne sec et 33 g (0,84 at.g) de potassium coupé en dés. (1) introduit dans l'ampoule (94,2 g (0,42 mole)) est coulé goutte à goutte dans le ballon à température ambiante. On note une réaction exothermique au cours de la coulée et l'apparition d'un précipité beige. En fin de coulée (2 h), la température dans le ballon a atteint 30°C. Le milieu est maintenu sous agitation jusqu'à disparition totale du potassium (après 12 h).

On coule alors dans le ballon placé dans un bain de glace 100 ml d'éthanol absolu. Le milieu est maintenu 1 heure sous agitation puis on coule 200 ml d'eau. Les solvants sont alors éliminés sous vide et le résidu est extrait par 300 ml d'éther. Les phases éthérées sont lavées avec 200 ml d'eau, séchées sur sulfate de magnésium puis concentrées.

Le liquide jaune clair obtenu est distillé au four à boules. On recueille (2) sous la forme d'un liquide incolore qui présente une température d'ébullition de 125°C sous 1,4 torr

Masse obtenue : 76 g Rendement : 86 %

Exemple 3

Préparation de la 2,6-dimethoxy 4-terbutyl acétophénone

On utilise deux ballons tricols de 250 ml munis d'un thermomètre et d'une ampoule de coulée isobare. Chacun d'entre eux est équipé d'un agitateur magnétique.
Dans le premier ballon, on introduit 1,5 g (11 mole) de $AlCl_3$ et 30 ml de $CH_2Cl_2$. Le ballon est refroidi à 0°C et l'on ajoute goutte à goutte 0,94 g (12 mmoles) de chlorure d'acétyle dissout dans 10 ml de $CH_2Cl_2$.
Le milieu homogène se colore en jaune pâle et on le maintient 1 h entre 0 et 5°C.
Dans le second ballon, on introduit 1,94 g (10 mmoles) de 1,3-diméthoxy 5-terbutyl benzène (2) dans 30 ml de $CH_2Cl_2$ puis on refroidit à 0°C.
Le complexe préparé précédemment est introduit dans l'ampoule de coulée à double enveloppe à 0°C puis coulé goutte à goutte dans le ballon.
On laisse réchauffer à température ambiante puis on maintient l'agitation pendant 10 h.
Le milieu est alors hydrolysé avec 100 ml d'eau à 5°C. Après décantation, la phase aqueuse est extraite avec 3 fois 20 ml de $CH_2Cl_2$ puis les phases organiques lavées avec 2 fois 40 ml d'eau.
Après séchage sur sulfate de magnésium et concentration on recueille un miel marron clair qui est chromatographié sur silice (éluant : Heptane/acétate d'éthyle : 90/10).
On recueille (3) sous la forme de cristaux blancs fondant à 57,2°C
Masse obtenue : 1,67 g          Rendement : 71 %

TABLEAU I

Composés répondant à la formule générale

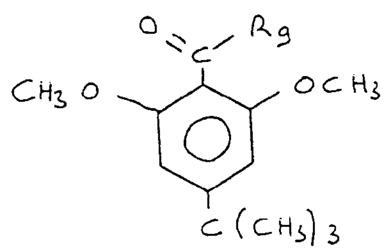

| Composé n° | R | Caractéristiques |
|---|---|---|
| 3 | $CH_3$ | F = 57,2°C |
| 4 | $C_2H_5$ | F = 60,2°C |
| 5 | $nC_3H_7$ | miel |
| 6 | $iC_3H_7$ | F = 56,8°C |
| 7 | cyclopropyle | F = 71,5°C |
| 8 | $(CH_2)_2-Cl$ | F = 76 °C |
| 9* | $(CH_2)_2N(CH_3)_2$ | Miel |
| 10* | $(CH_2)_2$ $NH(CH_3)_2Cl$ | F = 180° C |
| 11* | $(CH_2)_2$ $SCH_3$ | miel |

* Les composés (9), (10) et (11) ont été préparés à partir du dérivé (8) selon des méthodes bien connues.

On donne ci-après la nomenclature des composés 3 à 11 :

3 2,6-diméthoxy 4-terbutyl acétophénone

4 2,6-diméthoxy 4-terbutyl propiophénone

5 2,6-diméthoxy 4-terbutyl butyrophénone

6 (2,6-diméthoxy 4-terbutyl phényl) isopropyl cétone

7 (2,6-diméthoxy 4-terbutyl phényl) cyclopropyl cétone

8 (2,6-diméthoxy 4-terbutyl phényl) 2-chloroéthyl cétone

9 (2,6-diméthoxy 4-terbutyl phényl) 2-diméthylamino éthyl cétone

10 (2,6-diméthoxy 4-terbutyl phényl) 2-diméthylamino éthyl cétone chlorydrate

11 (2,6-diméthoxy 4-terbutyl phényl) 2-méthyl thioéthyl cétone.

Exemple (12)

Préparation du (2,6-diméthoxy 4-terbutylphényl)-1 méthanol

On utilise un ballon tricol muni d'un thermomètre, d'un réfrigérant, d'une ampoule de coulée isobare et parcouru par un courant d'azote sec. L'agitation est assurée par un agitateur magnétique.

Dans le ballon, on introduit 30 ml d'éther éthylique sec puis 0,42 g (11 mmoles) d'hydrure de lithium aluminium. Sur cette suspension refroidie à 10°C, on coule goutte à goutte 2,36 g (10 mmoles) de 2,6-diméthoxy 4-terbutylacétophénone diluée dans 10 ml d'éther.

La température atteint 13°C en fin de coulée ; on laisse revenir à température ambiante puis on maintient le milieu sous agitation encore 30 mn. L'excès d'hydrure est ensuite détruit à 5°C en coulant goutte à goutte 2 ml d'acétate d'éthyle dans le ballon. Le milieu est hydrolysé avec 30 ml d'une solution HCl 0,1 N. La phase éthérée est lavée avec 3 x 30 ml d'eau, séchée sur Mg $SO_4$ puis concentrée. On recueille (12) sous la forme d'une poudre blanche fondant à 48,2°C.

Masse obtenue 2,33 g Rendement : 98 %

TABLEAU II

Composés répondant à la formule générale

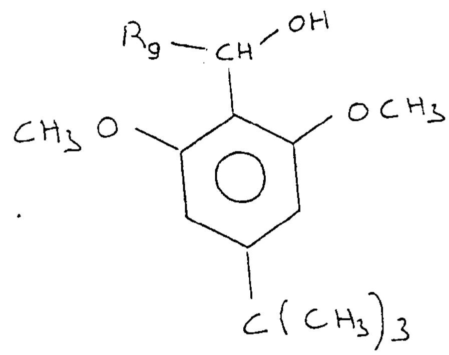

| Composé n° | $R_9$ | Caractéristiques |
|---|---|---|
| 12 | $CH_3$ | F = 48,2°C |
| 13 | $C_2H_5$ | F = 30,6°C |
| 14 | $nC_3H_7$ | miel |
| 15 | $iC_3H_7$ | F = 56,8°C |
| 16 | cyclopropyl | F = 60,4°C |
| 17* | 3-pyridinyl | miel |

Exemple (17)

Préparation du (2,6-diméthoxy 4-terbutylphényl) pyridinyl-3 méthanol

On utilise un ballon tricol de 250 ml muni d'un thermomètre, d'un réfrigérant, d'une ampoule de coulée isobare, le montage étant parcouru par un courant d'azote sec. L'agitation est assurée par un agitateur magnétique. Dans le tricol, on introduit sous azote 4 g(20 mmoles) de 1,3-diméthoxy 5-terbutylbenzène et 40 ml d'éther éthylique.

On coule sur cette solution 14 ml (22,6 mmoles) de n-Buthyllithium 1,6 M à température ambiante. La température s'élève de 23°C à 27°C en fin de coulée. La solution est portée au reflux de l'éther pendant 5 h. Elle prend une coloration jaune clair.

On laisse la température revenir à l'ambiante puis on coule 2,42 g (22,6 mmoles) de pyridine-3 carboxaldéhyde dilué dans 10 ml d'éther. La température s'élève de 22°C à 25°C.

Le milieu est ensuite maintenu 2 h sous agitation à température ambiante avant d'être hydrolysé avec 60 ml d'eau, extrait avec 2 x 30 ml d'éther, lavé avec 3 x 40 ml d'eau, séché sur Mg $SO_4$ puis concentré.

On recueille 7,5 g d'un miel qui est chromatographié sur colonne de silice (éluant : acétate d'éthyle). (17) est obtenu sous la forme d'un miel épais.

Masse obtenue : 4,8 g Rendement : 77 %

On donne ci-après la nomenclature des composés 12 à 17.

12 (2,6-diméthoxy 4-terbutylphényl)-1-méthanol

13 (2,6-diméthoxy 4-terbutylphényl)-1-éthanol

14 (2,6-diméthoxy 4-terbutylphényl)-1-propanol

15 (2,6-diméthoxy 4-terbutylphényl)-1-propanol 2-méthyl

16 (2,6-diméthoxy 4-terbutylphényl) cyclopropyl méthanol
17 (2,6-diméthoxy 4-terbutylphényl) pyridinyl-3 méthanol.

Exemple (18)

Préparation de l'acide 2,6-diméthoxy 4-terbutyl benzoïque

L'anion lithié du 1,3-diméthoxy 5-terbutylbenzène est préparé de la même manière que dans l'exemple (17) et dans les mêmes proportions.
Sur le sel formé, on fait buller un excès d'anhydride carbonique à température ambiante puis on maintient l'agitation encore 1 h. Le sel de l'acide formé précipite partiellement dans le milieu. On l'hydrolyse avec 40 ml d'eau avant de l'extraire avec 3 x 30 ml d'éther.
La phase aqueuse est reprise puis traitée avec 30 ml d'HCl 1 N. L'acide libéré précipite dans le milieu.
Il est filtré, lavé avec 3 x 30 ml d'eau puis séché sous vide au dessicateur chauffant. On recueille (18) sous la forme d'une poudre blanche fondant à 158°C.
Masse recueillie : 3,57 g    Rendement : 75 %
Les dérivés de l'acide sont préparés à partir de celui-ci selon les méthodes connues de la littérature.

TABLEAU III

Composé répondant à la formule générale

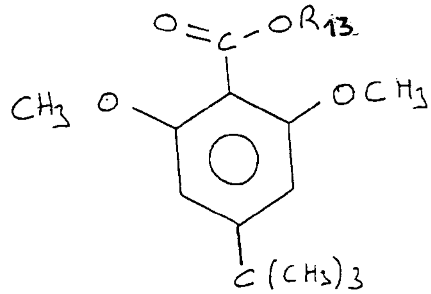

| Composé n° | $R_{13}$ | Caractéristiques |
|---|---|---|
| 18 | H | F = 158°C |
| 19 | Na | F > 300°C |
| 20 | $CH_3$ | F = 98°C |
| 21 | $C_2H_5$ | F = 73,3°C |

On donne ci-après la nomenclature des composés 18 à 21.
(18) acide 2,6-diméthoxy 4-terbutyl benzoïque
(19) 2,6-diméthoxy 4-terbutyl benzoate de sodium
(20) 2,6-diméthoxy 4-terbutylbenzoate de méthyle
(21) 2,6-diméthoxy 4-terbutylbenzoate d'éthyle

Exemple (22)

Préparation du (2,6-diméthoxy 4-terbutylphényl)-1-acyloxy propane

On utilise un ballon bicol muni d'un thermomètre et d'une ampoule de coulée isobare. L'agitation est assurée par un agitateur magnétique.
Dans le ballon, on introduit 1 g (3,9 mmoles) de (2,6-diméthoxy 4-terbutylphényl)-1-éthanol (13), 0,35 g(4,5

mmoles) de pyridine et 15 ml d'éther. On coule goutte à goutte dans le ballon 0,32 g (4,1 mmoles) de chlorure d'acéthyle dilué dans 10 ml d'éther à température ambiante. Un précipité blanc de chlorydrate de pyridinium apparaît rapidement.

L'agitation est maintenue à température ambiante pendant 3 heures. Le milieu est ensuite hydrolysé avec 25 ml d'eau. La phase aqueuse est extraite avec 2 x 20 ml d'éther, les phases éthérées rassemblées sont lavées avec 3 x 25 ml d'eau séchées sur Mg $SO_4$ puis concentrées.

(22) est recueilli sous la forme d'une poudre blanche fondant à 60°C (pentane).

Masse recueillie : 0,9 g Rendement : 79 %

TABLEAU IV

Composé répondant à la formule

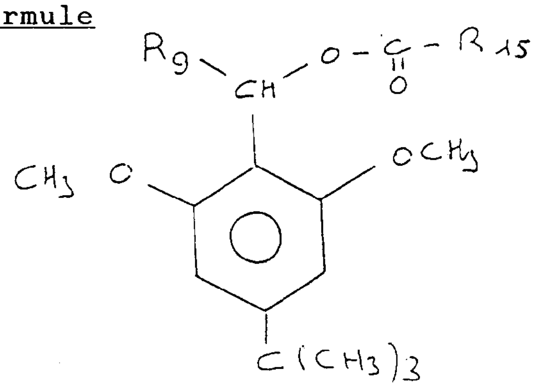

| Composé n° | R 9 | $R_{15}$ | Caractéristiques |
|---|---|---|---|
| 22 | $C_2H_5$ | $CH_3$ | F = 60°C |
| 23 | $nC_3H_7$ | $CH_3$ | F = 48°C |
| 24 | cyclopropyle | $CH_3$ | F = 76°C |
| 25 | $C_2H_5$ | Ph | miel |
| 26 | $C_2H_5$ | $CH_2CH(CH_3)_2$ | miel |
| 27 | $C_2H_5$ | $C(CH_3)_3$ | F = 52°C |

On donne ci-après la nomenclature des composés 22 à 27.

22 (2,6-diméthoxy 4-terbutylphényl)-1-acyloxy propane

23 (2,6-diméthoxy 4-terbutylphényl)-1-acyloxybutane

24 (2,6-diméthoxy 4-terbutylphényl)-1-acyloxy méthylcyclopropane

25 (2,6-diméthoxy 4-terbutylphényl)-1-benzoyloxypropane

26 (2,6-diméthoxy 4-terbutylphényl)-1-isobutyryl carbonyl oxypropane

27 (2,6-diméthoxy 4-terbutylphenyl)-1-terbutyl carbonyl oxypropane

Exemple 28

Préparation de la 2,6-diméthoxy 3-nitro 4-terbutyl propiophénone

Dans un ballon tricol de 250 ml équipé d'une agitation mécanique et d'un réfrigérant, on introduit 90 ml d'$H_2SO_4$ à 95 % puis on refroidit à -12°C. On ajoute alors 18,1 g (0,072 mole) de 2,6-diméthoxy 4-terbutyl propiophénone (cf exemple 4), puis on introduit par petites fractions 7,7 g (0,076 mole) de nitrite de potassium. La réaction est exothermique, la température montant à -7° C en fin d'ajout (10mn). Le mélange réactionnel est maintenu 3 heures sous agitation à 0° C. La solution est versée sur 1 l d'eau glacée, extraite par 3 x 80ml d'éther. Les

phases organiques rassemblées sont lavées à l'eau jusqu'à neutralité, séchées sur sulfate de sodium puis concentrées. On obtient une huile orangée qui est chromatographiée sur colonne de silice (éluant $CH_2CL_2$/heptane : 50/50). On obtient le composé 28 souhaité sous la forme d'une poudre jaune clair fondant à 45°C.
Masse recueillie : 13 g    Rendement : 61 %

Selon le même procédé les composés suivants ont été obtenus.

TABLEAU V

Composé répondant à la formule

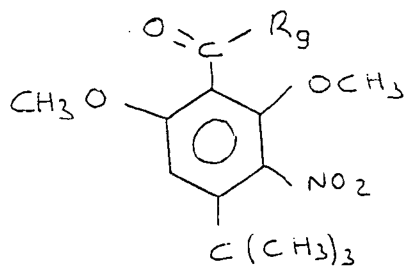

| Composé n° | R | Caractéristiques |
|---|---|---|
| 29 | $iC_3H_7$ | F = 60,2°C |
| 30 | cyclopropyle | F = 80,1°C |
| 31 | $OCH_3$ | F = 90°C |
| 32 | $OC_2H_5$ | F = 69°C |

On donne ci-après la nomenclature des composés 29 à 32 :
29 (2,6-diméthoxy 3-nitro 4-terbutylphényl) isopropyl cétone
30 (2,6-diméthoxy 3-nitro 4-terbutylphényl) cyclopropyl cétone
31 2,6-diméthoxy 3-nitro 4-terbutylbenzoate de méthyle
32 2,6-diméthoxy 3-nitro 4-terbutylbenzoate d'éthyle.

Exemple 33

Préparation du (2,6-diméthoxy 3-nitro 4-terbutylphényl)-1-propanol

Dans un ballon tricol de 100 ml équipé d'une ampoule de coulée, et d'un réfrigérant, on introduit 20 ml de THF sec puis 0,2 g (0,005 mole) d'hydrure de lithium aluminium. Le milieu est refroidi à 0° C. On coule sous agitation magnétique le composé de l'exemple 28 dilué dans 15 ml de THF. La température s'élève de 5° C. On agite 2 heures à 10° C puis on ajoute successivement 5 ml d'acétate d'éthyle, 1 ml d'eau, 1 ml d'une solution à 15% en NaOH puis 3 ml d'eau. Le précipité blanc formé est filtré et le filtrat lavé avec 3 x 20 ml d'eau, séché sur sulfate de sodium puis concentré.
On obtient le composé 33 souhaité sous la forme d'une huile jaune.
Masse receuillie : 1,3 g    Rendement : 86 %

Selon le même procédé ont été obtenus les composés réunis dans le tableau suivant :

TABLEAU VI

Composé répondant à la formule

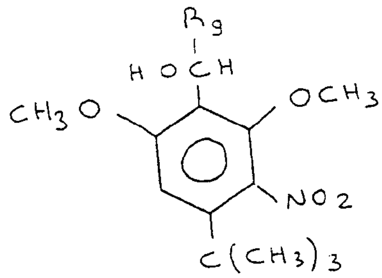

| Composé n° | R | Caractéristiques |
|---|---|---|
| 34 | $iC_3H_7$ | miel |
| 35 | cyclopropyle | miel |

On donne ci-après la nomenclature des composés 34 et 35
34 (2,6-diméthoxy 3-nitro 4-terbutylphényl)- 1 propanol 2-méthyl
35 (2,6-diméthoxy 3-nitro 4-terbutylphényl) cyclopropyl méthanol

Exemple 36

Préparation de la 2,4-diméthoxy 3-(1-oxo propyl) 6-terbutyl aniline

Dans un ballon tricol de 1000 ml équipé d'une agitation mécanique et d'un réfrigérant, on introduit 13,1 g (0,044 mole) du composé de l'exemple 28, 23 ml (0,4 mole) d'acide acétique glacial et 130 ml d'éthanol absolu. On porte à reflux puis on ajoute par portions en 45 minutes 10,45 g (0,18 mole) de fer en poudre. Le milieu est porté à reflux pendant encore 10 heures. On verse le contenu sur 600 ml d'eau avant d'extraire à l'éther, de sécher la phase organique sur sulfate de sodium puis de concentrer.
On obtient 13 g d'une huile rouge qui est chromatographiée sur colonne de silice. Eluant $CH_2Cl_2$/heptane : 50/50.
On obtient le composé 36 souhaité sous la forme d'un solide blanc fondant à 62° C.
Masse recueillie : 6,5 g Rendement : 65,2 %

Exemple 37

Préparation de la 2,4-diméthoxy 3-(1-hydroxypropyl) 6-terbutyl aniline

Le protocole opératoire est identique à celui décrit dans l'exemple 33 à partir du composé de l'exemple 36.
Le composé 37 souhaité est obtenu sous la forme d'une huile brune.
Masse recueillie : 1,2 g Rendement : 98 %

Exemple 38

Préparation du chlorydrate de la 2,4-diméthoxy 3-(1-oxopropyl) 6-terbutyl aniline

Dans un tricol de 100 ml, on introduit 0,4 g (0,0015 mole) du composé n° 36 puis 15 ml d'éther. On fait buller un courant d'HCl gaz (2 g soit 0,055 mole) en 15 mn. Le précipité blanc formé est filtré, lavé à l'éther puis séché sous vide.
On obtient le composé 38 souhaité sous la forme d'un solide blanc fondant à 110° C.
Masse recueillie : 0,45 g Rendement : 99 %

L'invention concerne également l'utilisation à titre d'herbicide des composés de formule (I). Comme mauvaises herbes pouvant être contrôlées ou détruites par les composés de formule (I), on peut citer :

| Abréviations | Nom latin | Nom français |
|---|---|---|
| AVEFA | Avena fatua | Folle avoine |
| ECHCG | Echinochloa crus-galli | Panisse |
| ALOMY | Alopecurus myosuroïdes | Vulpin |
| DIGSA | Digitaria sanguinalis | Digitaire |
| SETFA | Setaria faberi | Sétaire |
| SOLNI | Solanum nigrum | Morelle |

L'utilisation des composés de formule (I) est la plupart du temps sous forme de composition herbicide comportant un ou plusieurs supports acceptables en agriculture.

En effet pour leur emploi pratique, les composés selon l'invention sont rarement utilisés seuls. Le plus souvent ces composés font partie de compositions. Ces compositions, utilisables comme agents herbicides, contiennent comme matière active un composé selon l'invention tel que décrit précédemment en mélange avec les supports solides ou liquides, acceptables en agriculture et les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention, un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol etc...).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de trés larges limites, allant de 0,05 % à 95 % (en poids). Leur teneur en agent tensio-actif est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas).

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnalbes :

<u>Exemple F 1</u> :

| | |
|---|---|
| - matière active ........................ | 400 g/l |
| - dodécylbenzène sulfonate alcalin ....... | 24 g/l |
| - nonylphénol oxyéthylé à 10 molécules d'oxyde d'éthylène .................... | 16 g/l |
| - cyclohexanone ......................... | 200 g/l |
| - solvant aromatique ...............q.s.p. | 1 litre |

Selon une autre formule de concentré émulsionnable, on utilise :

<u>Exemple F 2</u>

| | |
|---|---|
| - matière active ...................... | 250 g |
| - huile végétale époxydée .............. | 25 g |
| - mélange de sulfonate d'alcoylaryle et d'éther de polyglycol et d'alcools gras | 100 g |
| - diméthylformamide .................... | 50 g |
| - xylène ............................... | 575 g |

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

Exemple F 3 :

| | |
|---|---|
| - composé ................................ | 500 g |
| - phosphate de tristyrylphénol polyéthoxylé | 50 g |
| - alkylphénol polyéthoxylé ............... | 50 g |
| - polycarboxylate de sodium .............. | 20 g |
| - éthylène glycol ....................... | 50 g |
| - huile organopolysiloxanique (antimousse) ... | 1 g |
| - polysaccharide .......................... | 1,5 g |
| - eau .................................. | 316,5 g |

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0,1 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables trés avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

Exemple F 4

| | |
|---|---|
| - matière active (composé de formule I)..... | 50 % |
| - alcool gras éthoxylé (agent mouillant)..... | 2,5 % |
| - phényléthylphénol éthoxylé (agent dispersant | 5 % |
| - craie (support inerte).................... | 42,5 % |

Exemple F 5

| | |
|---|---|
| - matière active (composé de formule I...... | 10 % |
| - alcool synthétique oxo de type ramifié, en C13 éthoxylé par 8 à 10 oxyde d'éthylène (agent mouillant)...................... | 0,75 % |
| - lignosulfonate de calcium neutre (agent dispersant)........................... | 12 % |
| - carbonate de calcium (charge inerte) .q.s.p. | 100 % |

Exemple F 6

Cette poudre mouillable contient les mêmes ingrédients que dans l'exemple précédent, dans les proportions ci-après :

- matière active ........................ 75 %
- agent mouillant ....................... 1,50 %
- agent dispersant ...................... 8 %
- carbonate de calcium (charge inerte) q.s.p. 100 %

Exemple F 7

- matière active (composé de formule I).... 90 %
- alcool gras éthoxylé (agent mouillant) .. 4 %
- phényléthylphénol éthoxylé (agent dispersant) 6 %

Exemple F 8

- matière active (composé de formule I).... 50 %
- mélange de tensio-actifs anioniques et non ioniques (agent mouillant)........... 2,5 %
- lignosulfonate de sodium (agent dispersant) 5 %
- argile kaolinique (support inerte) ....... 42,5 %

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple F 9 : Granulés dispersibles

Dans un mélangeur, on mélange 90 % en poids de matière active (composé de formule I) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple F 10 : Granulés dispersibles

Dans un mélangeur, on mélange les constituants suivants :

- matière active (composé de formule I) .... 75 %
- agent mouillant (alkylnaphtalène sulfonate de sodium ................................ 2 %
- agent dispersant (polynaphtalène sulfonate de sodium) ............................ 8 %
- charge inerte insoluble dans l'eau (kaolin) 15 %

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

En ce qui concerne les compositions adaptées au stockage et au transport, elles contiennent plus avantageusement de 0,5 à 95 % (en poids) de substance active.

La présente invention concerne aussi un procédé de désherbage (notamment de zones de culture dicotylédones ou monocotylédones) qui consiste à appliquer aux plantes devant être détruites ou sur le sol une quantité efficace d'un composé de formule (I).

On utilisera, de préférence, un procédé de désherbage qui consiste à appliquer une quantité efficace d'un composé de formule (I) sur des zones ou terrains où l'on veut empêcher la pousse ou le développement de plantes n'ayant pas encore poussé (application de préémergence).

On peut opérer de manière à ce que la culture soit semée avant ou après traitement.

La dose d'application de matière active est généralement comprise entre 1 et 8 000 g/ha.

Les exemples ci-dessous illustrent l'invention :

Exemple A - Application herbicide, en prélevée des espèces végétales

Dans des pots de 7 x 7 x 8 cm remplis de terre agricole légère, on sème un nombre de graines déterminé en fonction de l'espèce végétale et de la grosseur de la graine.

Les pots sont traités par pulvérisation de bouillie en quantité correspondant à une dose volumique d'application de 500 l/ha et contenant la matière active à la concentration désirée.

Le traitement avec la bouillie est donc effectué sur des graines non recouvertes de terre (on utilise le terme de bouillie pour désigner, en général, les compositions diluées à l'eau, telles qu'on les applique sur les végétaux).

La bouillie utilisée pour le traitement est une solution ou suspension de la matière active dans un mélange acétone/eau en proportions 50/50, en présence de 0,05 % en poids de cemulsol NP 10 (agent tensio-actif) constitué d'alkylphénol polyéthoxylé, notamment de nonylphénol polyéthoxylé) et 0,04 % en poids de tween 20 (agent tensio-actif constitué d'un oléate de dérivé polyoxyéthyléné du sorbitol).

Dans le cas d'une suspension, celle-ci est obtenue en mélangeant et broyant les ingrédients dans un microniseur de façon à obtenir une grosseur moyenne de particules inférieure à 40 microns.

Après traitement, on recouvre les graines d'une couche de terre d'environ 3 mm d'épaisseur.

Les pots sont alors placés dans des bacs destinés à recevoir l'eau d'arrosage, en subirrigation, et maintenus pendant 24 jours à température ambiante sous 40 à 60 % d'humidité relative.

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage de volume foliaire détruit et réduit par le produit est donc donné par la formule.

$$D + \frac{RT\,(100 - D)}{100} = A$$

Cette valeur A est transformée en notation de 0 à 5 selon l'échelle suivante :

| | notation | |
|---|---|---|
| 0 < A < 10 | 0 | pas d'effet |
| 10 < A < 30 | 1 | |
| 30 < A < 50 | 2 | |
| 50 < A < 70 | 3 | |
| 70 < A < 90 | 4 | |
| 90 < A < 100 | 5 | destruction complète |

Les résultats obtenus sont présentés pour des doses d'application de 4000 g/ha.

| Abréviations | Nom latin | Nom français |
|---|---|---|
| AVEFA | Avena fatua | Folle avoine |
| ECHCG | Echinochloa crus-galli | Panisse |
| DIGSA | Digitaria sanguinalis | Digitaire |
| ALOMY | Alopecurus myosuroides | Vulpin |
| SOLNI | Solanum nigrum | Morelle |

| COMPOSE N° | ACTIVITE HERBICIDE DE PRELEVEE Dose : 4 kg m.a./ha | | | | |
|---|---|---|---|---|---|
| | AVEFA | ECHCG | ALOMY | DIGSA | SOLNI |
| 6 | 5 | 5 | 5 | 5 | 4 |
| 7 | 5 | 5 | 5 | 5 | 4 |
| 12 | 4 | 5 | 5 | 5 | 4 |
| 13 | 5 | 5 | 5 | 5 | 5 |
| 15 | 5 | 5 | 5 | 5 | 5 |
| 16 | 5 | 5 | 5 | 5 | 4 |
| 22 | 5 | . | 5 | 5 | 5 |
| 23 | 4 | . | 5 | 5 | 3 |
| 25 | 5 | . | 5 | 5 | 4 |
| 27 | 5 | 5 | 5 | 5 | 4 |

| Dose : 2 kg m.a./ha | | | | | |
|---|---|---|---|---|---|
| 28 | 1 | 4 | 3 | 5 | 3 |
| 29 | 0 | 0 | 0 | 4 | 1 |
| 30 | 1 | 4 | 4 | 5 | 3 |
| 31 | 1 | 4 | 4 | 5 | 3 |

Exemple B - Essai de sélectivité en grandes cultures avec application herbicide, en prélevée des espèces végétales.

Dans des pots de 7 x 7 x 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur.

Les pots sont alors traités par pulvérisation de bouillie en quantité correspondant à une dose volumique d'application de 500 l/ha et contenant la matière active à la concentration désirée.

La bouillie a été préparée de la même manière qu'à l'exemple A.

Les pots traités sont ensuite placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage de volume foliaire détruit-réduit par le produit est donc donné par la formule :

$$D + \frac{RT\,(100 - D)}{100} = A$$

Cette valeur A est transformée en notation de 0 à 5 selon l'échelle suivante :

| | notation | |
|---|---|---|
| 0 < A < 10 | 0 | pas d'effet |
| 10 < A < 30 | 1 | |
| 30 < A < 50 | 2 | |
| 50 < A < 70 | 3 | |
| 70 < A < 90 | 4 | |
| 90 < A < 100 | 5 | destruction complète |

Ainsi, un produit est jugé comme sélectif vis à vis de la culture lorsque la valeur A notée est 0 ou 1.

Les résultats obtenus sont présentés dans l'exemple B pour des doses d'application de 2 ou 4 kg de matière active par hectare selon les produits. Les espèces végétales utilisées dans cet exemple sont :

1) Pour les adventices

| Abréviations | Nom latin | Nom français |
|---|---|---|
| ECHCG | Echinochloa crus-galli | Panisse |
| DIGSA | Digitaria sanguinalis | Digitaire |
| SETFA | Setaria Faberi | Setaire |

2) Pour les cultures

| Abréviations | Nom latin | Nom français |
|---|---|---|
| TRZAS | Triticum aestivum | Blé |
| ZEAMX | Zea mays | Maïs |
| ORYSA | Oryza sativa | Riz |
| GLXMA | Glycine maximum | Soja |
| GOSHI | Gossypium hirsutum | Coton |
| HELAN | Helianthus annuus | Tournesol |

## ESSAI DE SELECTIVITE EN GRANDES CULTURES
## ACTIVITE HERBICIDE DE PRELEVEE

| Composé n° | Dose appliquée kg/ha | ECH | DIG | SET | TRZ | ZEA | ORY | GLX | GOS | HEL |
|---|---|---|---|---|---|---|---|---|---|---|
| 4 | 4 | 5 | 5 | 5 | 1 | 3 | 1 | 0 | 0 | 0 |
| 6 | 2 | 5 | 5 | 3 | 0 | 0 | 0 | 1 | 0 | 1 |
| 7 | 2 | 5 | 5 | 5 | 0 | 0 | 0 | 0 | 0 | 0 |
| 13 | 2 | 5 | 5 | 5 | 0 | 0 | 0 | 0 | 1 | 0 |
| 15 | 2 | 5 | 5 | 3 | 0 | 0 | 1 | 0 | 0 | 0 |
| 16 | 4 | 5 | 5 | 3 | 0 | 0 | 0 | 0 | 0 | 0 |
| 20 | 4 | 5 | 5 | 4 | 0 | 1 | 0 | 0 | 1 | 0 |
| 24 | 2 | 5 | 5 | 4 | 0 | 1 | 1 | 0 | . | . |
| 28 | 2 | 4 | 5 | 4 | 0 | 0 | 0 | 0 | . | . |
| 29 | 2 | 0 | 4 | 3 | 0 | 0 | 0 | 0 | . | . |
| 30 | 2 | 4 | 5 | 5 | 0 | 0 | 0 | 0 | . | . |
| 31 | 2 | 4 | 5 | 4 | 0 | 0 | 0 | 0 | . | . |

Comme on peut le voir sur le tableau de résultats de cet exemple B, de nombreux produits présentent une excellente activité antigramminée de prélevée tout en montrant une excellente sélectivité pour 1 ou 2 ou 3 ou 4 ou 5 ou 6 des 6 cultures testées = blé, maïs, riz, soja, coton, tournesol.

## Revendications

1. Composés de formule :

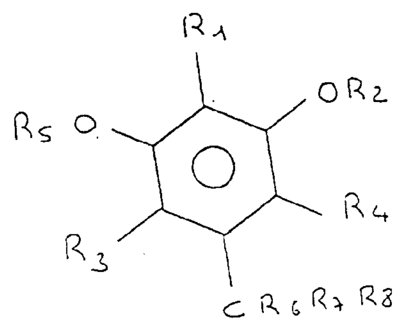

(I)

dans laquelle :
$R_1$ représente :

– un groupe -C(=X)-$R_9$, X = 0 ou S et $R_9$ étant un atome d'hydrogène ou un radical $C_1$-$C_6$ alkyle éventuellement substitué par un ou plusieurs atomes d'halogène ou radicaux $C_1$-$C_4$ alkoxy, $C_1$-$C_4$ alkylthio ; un radical $C_2$-$C_6$ alcynyle ou $C_2$-$C_6$ alcényle, lesdits radicaux étant éventuellement substitués de la même manière que le groupement $C_1$-$C_6$ alkyle ;un radical di ($C_1$-$C_3$) alkylamino ($C_1$-$C_6$) alkyl, di ($C_1$-$C_3$) alkylamino $C_1$-$C_6$ alkylènyle, amino $C_1$-$C_6$ alkylènyle ou mono ($C_1$-$C_3$) alkylamino $C_1$-$C_6$ alkylènyle ; un radical $C_3$-$C_6$ cycloalkyle substitué ou non par un ou plusieurs atomes d'halogène ou par un ou plusieurs radicaux choisis parmi les radicaux $C_1$-$C_6$ alkyle, $C_1$-$C_6$ haloalkyle, $C_1$-$C_6$ alkoxy, $C_1$-$C_6$ thioalkoxy ; un radical $C_6$-$C_{10}$ aryle substitué ou non par un ou plusieurs atomes d'halogène ou par un ou plusieurs radicaux choisis parmi les radicaux $C_1$-$C_6$ alkyle, $C_1$-$C_6$ haloalkyle, $C_1$-$C_6$ alkoxy, $C_1$-$C_6$ thioalkoxy ; un radical $C_7$-$C_{11}$ aralkyle éventuellement substitué de la même manière que le radical $C_6$-$C_{10}$ aryle ; un radical hétéroaryle ayant 5 ou 6 atomes dont 1 à 3 hétéroatomes choisis parmi le soufre, l'azote, l'oxygène, notamment les 2- ou 3-thinyle, les 2- ou 3- furyle, les pyrolyles, les pyrrazolyles, les pyridinyles, les imidazolyles, les triazolyles, les pyrimidinyles, les pyridazinyles, les 2-, 3-, 4-, 5-, oxazolyles ou thiazolyles,

– un groupe

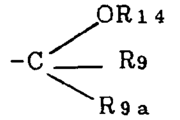

dans lequel $R_{9a}$ a l'une des significations mentionnées pour $R_9$, $R_{14}$ forme, ensemble avec $R_9$ ou $R_{9a}$, une chaîne $C_1$-$C_6$ alkylène ou $R_{14}$ est l'atome d'hydrogène, un radical $C_1$-$C_6$ alkyle éventuellement substitué par un ou plusieurs atomes d'halogène ou radicaux $C_1$-$C_4$ alkoxy, $C_1$-$C_4$ alkylthio ; un radical $C_2$-$C_6$ alcynyle ou $C_2$-$C_6$ alcényle lesdits radicaux étant éventuellement substitués de la même manière que le groupement $C_1$-$C_6$ alkyle ; un radical $C_3$-$C_6$ cycloalkyle substitué ou non par un ou plusieurs atomes d'halogène ou par un ou plusieurs radicaux choisis parmi les radicaux $C_1$-$C_6$ alkyle, $C_1$-$C_6$ haloalkyle, $C_1$-$C_6$ alkoxy, $C_1$-$C_6$ thioalkoxy ; un radical $C_6$-$C_{10}$ aryle substitué ou non par un ou plusieurs atomes d'halogène ou par un ou plusieurs radicaux choisis parmi les radicaux $C_1$-$C_6$ alkyle, $C_1$-$C_6$ haloalkyle, $C_1$-$C_6$ alkoxy, $C_1$-$C_6$ thioalkoxy ; un radical $C_7$-$C_{11}$ aralkyle éventuellement substitué de la même manière que le radical $C_6$-$C_{10}$ aryle ; un radical hétéroaryle ayant 5 ou 6 atomes dont 1 à 3 hétéroatomes choisis parmi le soufre, l'azote, l'oxygène, notamment les 2- ou 3- thiényle, les 2- ou 3- furyle, les pyrolyles, les pyrrazolyles, les pyridinyles, les imidazolyles, les triazolyles, les pyrimidinyles, les pyridazinyles, les 2-, 3-, 4-, 5-, oxazolyles ou thiazolyles ;

$R_{14}$ est encore un radical C(=O)-$R_{15}$ dans lequel $R_{15}$ représente une chaine $C_1$-$C_6$ alkyle, éventuellement substitué par un ou plusieurs atomes d'halogène ou radicaux $C_1$-$C_4$ alkoxy, $C_1$-$C_4$ alkylthio, $C_1$-$C_4$ alkylsulfonyle, $C_1$-$C_4$ alkylsulfinyle ; un radical $C_2$-$C_6$ alcynyle ou $C_2$-$C_6$ alcényle lesdits radicaux étant éventuellement substitués de la même manière que le groupement $C_1$-$C_6$ alkyle ; un radical $C_3$-$C_6$ cycloalkyle substitué ou non par un ou plusieurs atomes d'halogène ou par un ou plusieurs radicaux

choisis parmi les radicaux $C_1$-$C_6$ alkyle, $C_1$-$C_6$ haloalkyle, $C_1$-$C_6$ alkoxy, $C_1$-$C_6$ thioalkoxy ; un radical $C_6$-$C_{10}$ aryle substitué ou non par un ou plusieurs atomes d'halogène ou par un ou plusieurs radicaux choisis parmi les radicaux $C_1$-$C_6$ alkyle, $C_1$-$C_6$ haloalkyle, $C_1$-$C_6$ alkoxy, $C_1$-$C_6$ thioalkoxy ; un radical $C_7$-$C_{11}$ aralkyle éventuellement substitué de la même manière que le radical $C_6$-$C_{10}$ aryle ; un radical hétéroaryle ayant 5 ou 6 atomes dont 1 à 3 hétéroatomes choisis parmi le soufre, l'azote, l'oxygène, notamment les 2- ou 3- thiényle, les 2- ou 3- furyle, les pyrolyles, les pyrrazolyles, les pyridinyles, les imidazolyles, les triazolyles, les pyrimidinyles, les pyridazinyles, les 2-, 3-, 4-, 5-, oxazolyles ou thiazolyles,

– un groupe -C(=O)-$R_{16}$ dans lequel $R_{16}$ est $OR_{13}$ ou $SR_{13}$, avec $R_{13}$, qui peut être un atome d'hydrogène ou un radical $C_1$-$C_6$ alkyle, $C_2$-$C_6$ alcényle ou $C_2$-$C_6$ alcynyle, un radical $C_6$-$C_{10}$ aryle, $C_7$-$C_{11}$ aralkyle, lesdits radicaux étant substitués comme précédemment ou hétéroaryle ayant de 5 à 6 atomes dont 1 à 3 hétéroatomes choisis parmi l'azote, le soufre, l'oxygène,

$R_2$, $R_5$, identiques ou différents, représentent un groupe $C_1$-$C_6$ alkyle, $C_2$-$C_6$ alcènyle, $C_2$-$C_6$ alcynyle, ces deux radicaux étant éventuellement substitués de la même façon que le radical $C_1$-$C_6$ alkyle indiqué pour le radical $R_9$,

$R_3$, $R_4$, identiques ou différents, représentent l'atome d'hydrogène ou un groupe $C_1$-$C_6$ alkyle, un radical nitro ou amino à la condition que un seul des radicaux $R_3$ ou $R_4$ puissent signifier nitro ou amino simultanément,

$R_6$, $R_7$, $R_8$ identiques ou différents représentent un radical $C_1$-$C_6$ alkyle, un radical $C_3$-$C_7$ cycloalkyle, et les sels acceptables en agriculture de ces composés.

**2.** Composés selon la revendication 1, caractérisés en ce que $R_9$ est un radical $C_1$-$C_4$ alkyle ; trifluorométhyle ; cyclopropyle, substitué ou non par un ou plusieurs radicaux méthyle ; phényle, substitué ou non par un ou plusieurs radicaux méthyle ; benzyle ou phénéthyle.

**3.** Composés selon la revendication 1, caractérisés en ce que $R_{14}$ est un atome d'hydrogène ou un radical $C_1$-$C_4$ alkyle ; trifluorométhyle ; cyclopropyle, substitué ou non par un ou plusieurs radicaux méthyle ; phényle, substitué ou non par un ou plusieurs radicaux méthyle ; benzyle ou phénéthyle.

**4.** Composés selon la revendication 1, caractérisés en ce que lorsque $R_{14}$ est -$COR_{15}$, $R_{15}$ est un radical $C_1$-$C_4$ alkyle ; trifluorométhyle ; cyclopropyle, substitué ou non par un ou plusieurs radicaux méthyle ; phényle, substitué ou non par un ou plusieurs radicaux méthyle ; benzyle ou phénéthyle.

**5.** Composés selon la revendication 1, caractérisés en ce que $R_{13}$ est un radical phényle ou benzyle.

**6.** Composés selon la revendication 1, caractérisés en ce que le radical $R_{9a}$ est l'atome d'hydrogène.

**7.** Composés selon la revendication 1, caractérisés en ce que X est l'atome d'oxygène

**8.** Composés selon la revendication 1, caractérisés en ce que le radical $CR_6R_7R_8$ est le tertiobutyle

**9.** Composés selon la revendication 1, caractérisés en ce que $R_3$ et $R_4$ sont l'atome d'hydrogène ou l'un des groupes $R_3$ ou $R_4$ est l'atome d'hydrogène et l'autre le radical nitro.

**10.** Composés selon la revendication 1, caractérisés en ce que $R_2$ et $R_5$ identiques ou différents correspondent au radical $C_1$-$C_3$ alkyle

**11.** Composés selon la revendication 10, caractérisés en ce que $R_2$ et $R_6$ sont le radical méthyle

**12.** Composés selon les revendications 1 et 2, caractérisés en ce que $R_1$ est le radical C(=O)-$R_9$ dans lequel $R_9$ est un radical $C_1$-$C_4$ alkyle.

**13.** Composés selon les revendications 1, 2, 3 et 6, caractérisés en ce que $R_1$ est le radical $HCOHR_9$ dans lequel $R_9$ est un radical $C_1$-$C_4$ alkyle.

**14.** Composés selon les revendications 1, 2, 3 et 6, caractérisés en ce que $R_1$ est le radical -$HCOR_{14}R_9$, $R_9$ étant un radical $C_1$-$C_4$ alkyle, $R_{14}$ étant un radical $C_1$-$C_4$ alkyle

**15.** Composés selon les revendications 1, 4 et 6, caractérisés en ce que $R_1$ est le radical $HCR_9OC(=O)R_{15}$, $R_{15}$ étant un radical $C_1$-$C_4$ alkyle.

**16.** Composition herbicide comportant 0,05 à 95 % en poids d'une matière active selon l'une des revendications 1 à 15 en combinaison avec les supports solides ou liquides acceptables en agriculture et les agents tensio actif acceptables en agriculture.

**17.** Procédé de désherbage, notamment de zones de culture dicotylédone ou monocotylédone, qui consiste à appliquer aux plantes devant être détruites une quantité efficace d'un composé de formule (I) selon l'une des revendications 1 à 15, de préférence en pré émergence.

**18.** Composés de formule (II), (III), (IV) utiles notamment comme intermédiaires dans les procédés de synthèse des composés selon la revendication 1.

Office européen des brevets

# RAPPORT DE RECHERCHE EUROPEENNE

Numero de la demande

EP 91 42 0434

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | JOURNAL OF THE AMERICAN CHEMICAL SOCIETY<br>vol. 79, 1957, WASHINGTON DC US<br>pages 6030 - 6032;<br>R.C. FUSON ET AL.: 'Duryl 2,6-Disubstituted Phenyl Ketones.'<br>* Pages 6030, 6032 * | 1,2,7-11 | C07C49/84<br>C07C43/23<br>C07C69/007<br>C07C205/45<br>C07C225/22<br>C07C205/37<br>C07C205/60<br>A01N35/04<br>A01N31/16 |
| A | GB-A-1 586 466 (LILLY INDUSTRIES LTD.)<br>* revendications * | 1 | |
| A | FR-A-1 559 267 (KABUSHIKI KAISHA RICOH)<br>* revendications * | 1 | |
| A | FR-A-1 499 864 (FISONS PHARMACEUTICALS LTD)<br>* revendications * | 1 | |
| A | DE-A-1 930 328 (FARBWERKE HOECHST AG)<br>* revendications * | 1 | |
| | ----- | | DOMAINES TECHNIQUES RECHERCHES (Int. Cl.5 ) |
| | | | C07C |

Le présent rapport a été établi pour toutes les revendications

| Lieu de la recherche | Date d'achèvement de la recherche | Examinateur |
|---|---|---|
| LA HAYE | 13 MARS 1992 | BONNEVALLE E.I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)